(19) Europäisches Patentamt
European Patent Office
Office européen des brevets

(11) Publication number: **0 019 955**
**A1**

(12) **EUROPEAN PATENT APPLICATION**

(21) Application number: 80200412.7

(51) Int. Cl.³: **C 07 D 307/85**, A 61 K 31/34

(22) Date of filing: 02.05.80

(30) Priority: 16.05.79 US 39624
07.06.79 US 46592
07.06.79 US 46593

(43) Date of publication of application: 10.12.80
Bulletin 80/25

(84) Designated Contracting States: BE DE FR GB NL

(71) Applicant: SHELL INTERNATIONALE RESEARCH MAATSCHAPPIJ B.V., Carel van Bylandtlaan 30, NL-2596 HR DEN HAAG (NL)

(72) Inventor: Carr, John Bernard, 5211 Lodge Creek, Houston Texas 77066 (US)
Inventor: Witiak, Donald Theodore, 500 West 12th Avenue The Ohio State University, Columbus Ohio 43210 (US)
Inventor: Mersmann, Harry John, 412 Kavanagh Avenue, Modesto California 95350 (US)

(74) Representative: Keuzenkamp, Abraham et al, c/o Shell Internationale Research Maatschappij B.V. P.O. Box 302, NL-2501 CH 's-Gravenhage (NL)

# (54) Benzofurancarboxylic acid derivatives, their preparation and their inclusion in lipogenesis inhibiting compositions.

(57) This invention relates to benzofuran carboxylic acid derivatives.

There are provided benzofurancarboxylic acid derivatives of formula

(I)

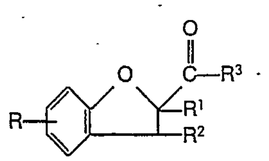

wherein R is a substituent at the 5-position on the benzofuran ring system and is a thienyl group, or a phenyl, phenoxy, benzyl or benzoyl group substituted by a group selected from alkoxy of 1 to 6 carbon atoms, hydroxy, acetyl, acetyloxy, 1-hydroxyethyl, acetamido and amino, or is a substituent at the 4-position of the benzofuran ring system and is a phenoxy group optionally substituted by a group selected from alkyl and alkoxy of 1 to 6 carbon atoms, hydroxy, acetyloxy, 1-hydroxyethyl, acetamido, amino, fluoro, chloro, bromo and nitro; $R^1$ and $R^2$ represent hydrogen atoms or $R^1$ and $R^2$ together represent a single chemical bond; and $R^3$ represents a hydroxy group, an alkoxy group of from 1 to 4 carbon atoms or a 2-propenyloxy group, or, when $R^1$ and $R^2$ represent hydrogen atoms, a 2-propenylamino group; processes for their preparation, lipogenesis inhibiting compositions containing them and the preparation of such compositions.

Compounds of formula I have exhibited lipogenesis inhibiting activity in mammalian tissue.

EP 0 019 955 A1

0019955

Benzofurancarboxylic acid derivatives, their preparation and their inclusion in lipogenesis inhibiting compositions

This invention relates to benzofurancarboxylic acid derivatives, to their preparation, and to their use as lipogenesis inhibitors in mammals.

Published UK Patent Application No. 2,007,973 relates to a broad class of benzofurancarboxylic acid derivatives which are described as having lipogenesis inhibiting activity in mammals. The synthesis of certain of those compounds was also described by Witiak, D.T., et al in Lipids, 11 (1976) (5), 384-391.

The applicants have now discovered a novel class of benzofurancarboxylic acid derivatives having lipogenesis inhibiting activity in mammals.

According to the invention there is provided a benzofurancarboxylic acid derivative of formula

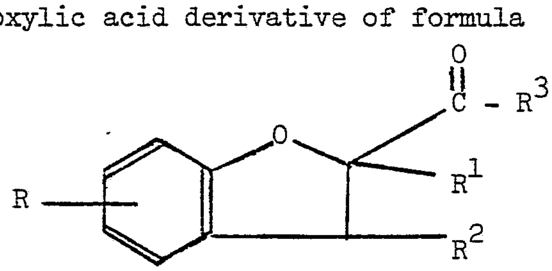

(I)

wherein R is a substituent at the 5-position on the benzofuran ring system and is a thienyl group, or a phenyl, phenoxy, benzyl or benzoyl group substituted by a group selected from alkoxy of 1 to 6 carbon atoms, hydroxy, acetyl, acetyloxy, 1-hydroxyethyl, acetamido and amino, or is a substituent at the 4-position of the benzofuran ring system and is a phenoxy group optionally substituted

by a group selected from alkyl and alkoxy of 1 to 6 carbon atoms, hydroxy, acetyl, acetyloxy, 1-hydroxyethyl, acetamido, amino, fluoro, chloro, bromo and nitro; $R^1$ and $R^2$ represent hydrogen atoms or $R^1$ and $R^2$ together represent a single chemical bond; and $R^3$ represents a hydroxy group, an alkoxy group of from 1 to 4 carbon atoms or a 2-propenyloxy group, or, when $R^1$ and $R^2$ represent hydrogen atoms, a 2-propenylamino group.

When $R^1$ and $R^2$ represent hydrogen atoms and $R^3$ represents a 2-propenylamino group, conveniently R is a substituent at the 5-position on the benzofuran ring system and is a thienyl group, or a phenyl, phenoxy, benzyl or benzoyl group substituted by an alkoxy group of 1 to 6 carbon atoms, a 1-hydroxyethyl group or an acetamido group, or is a substituent at the 4-position on the benzofuran ring system and is a phenoxy group optionally substituted by alkyl or alkoxy of 1 to 6 carbon atoms, acetamido, 1-hydroxyethyl or nitro.

When $R^3$ represents a hydroxy group, conveniently R is a substituent at the 5-position on the benzofuran ring system and is a phenyl, phenoxy, benzyl or benzoyl group substituted by a group selected from alkoxy of from 1 to 6 carbon atoms, acetyl, acetoxy, and hydroxy, or is a substituent at the 4-position on the benzofuran ring system and is a phenxoy group optionally substituted by a group selected from alkyl and alkoxy of 1 to 6 carbon atoms, fluorine, chlorine, bromine, nitro, acetyl, acetoxy and hydroxy. When $R^3$ represents an alkoxy group of from 1 to 4 carbon atoms or a 2-propenyloxy group, conveniently R represents a 5-(4-(1-hydroxyethyl)phenyl, 5-(4-acetamidophenyl) or 4-phenoxy group, or, when $R^1$ and $R^2$ together represent a single chemical bond, a 5-(4-acetylphenyl), 5-(4-acetyloxyphenyl) or 5-(4-aminophenyl) group.

When R is a substituted phenyl, phenoxy, benzyl or benzoyl group, the substituent is preferably attached at the 4-position thereof. An alkoxy substituent in R is preferably methoxy. R is preferably a substituted phenyl group attached at the 5-position of the benzofuran ring system or a phenoxy group attached at the 4-position of the benzofuran ring system.

In preferred compounds of formula I, R is a substituent at the 5-position on the benzofuran ring system and is a phenyl group substituted at the 4-position by an acetyl, acetyloxy, hydroxy, methoxy, 1-hydroxyethyl, acetamido or amino group or is a phenoxy group attached at the 4-position on the benzofuran ring system; Advantageously, R is a 5-(4-acetyloxyphenyl), 5-(4-(1-hydroxyethyl)phenyl), 5-(4-acetamidophenyl) or 4-phenoxy group.

It is further preferred in the compounds of formula I that $R^3$ represents a hydroxy, methoxy or ethoxy group or, when $R^1$ and $R^2$ represent hydrogen atoms, a 2-propenylamino group.

A particularly preferred group of compounds of formula I for their advantageous lipogenesis inhibiting activity in mammalian tissue is the group wherein $R^3$ is hydroxy, $R^1$ and $R^2$ together represent a single chemical bond and R is 4-phenoxy or 5-(4-acetyloxyphenyl); $R^3$ is methoxy or ethoxy and R is 5-(4-(1-hydroxyethyl)phenyl or 5-(4-acetamidophenyl; and $R^3$ is 2-propenylamino and R is 4-phenoxy or 5-(4-acetamidophenyl).

The invention also provides a process for preparing a benzofurancarboxylic acid derivative of formula I which process comprises reacting a salicylaldehyde of formula

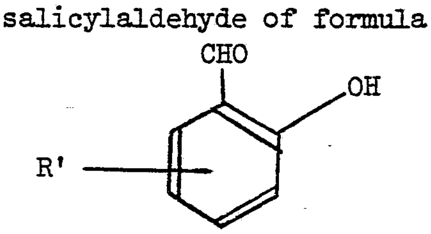

(II)

where R' is attached at the 5- or 6- position of the benzene ring and is R as defined above or a group which is convertible to R, with a halomalonate ester of formula

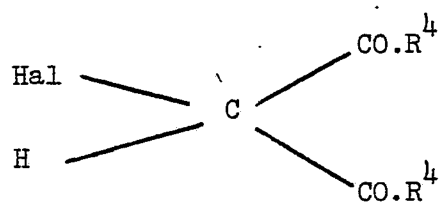

(III)

where Hal is bromine or chlorine and each $R^4$ is alkoxy of 1 to 4 carbon atoms in the presence of a base to give a benzofurancarboxylate ester of formula

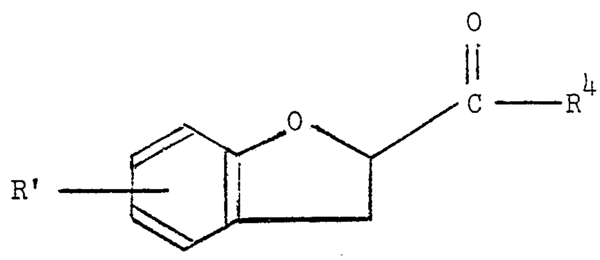

(IV)

followed, where necessary, by conversion of R' to R, reduction with sodium amalgam and/or conversion of $R^4$ to $R^3$.

Conveniently Hal in the compound of formula III is bromine. The preferred compound of formula III is diethyl bromomalonate. The reaction of the compounds of formula II and III is conveniently effected using an alkali metal carbonate, e.g. potassium carbonate, in anhydrous conditions in an inert solvent such as 2-butanone. Suitable conditions for this type of reaction are disclosed by Kurkudar and Rao, Indian Acad. Sci., Section A, 58, 336 (1963).

The precursor R'-subsituted salicylaldehydes of formula II can be prepared by treating the appropriate phenol with chloroform under strongly basic conditions, according to the Reimer-Tiemann Reaction. (References cited in The Merck Index, 9th edition, page ONR-74; also, Russell, A. and Lockhart. L.B., Organic Synthesis, 22, 63 (1942)).

Many of the precursor phenols are known compounds; others can be prepared by conventional procedures. For example, alpha-(4-hydroxyphenyl)thiophene, the precursor phenol for the compound of formula I wherein R is a 5-(2-thienyl) group can be prepared by either or both of the methods disclosed by M.A. Al'perovich, et al, Zh. Obschch, Khim., 34, 645-50 (1964); Chem. Abst., 60, 14639d (1964)), and Y. Ahmad, et al. Canadian Journal of Chemistry, 45, 1539-42 (1967).

R' is converted where necessary to an R group by known methods. When the R' group in the compound of formula II is different from the R group in the eventual compound of formula I, it may be another R group as defined above, or an unsubstituted phenyl, phenoxy, benzyl or benzoyl group.

Thus for example, if the starting compound of formula II is 5-phenylsalicylaldehyde, the R' group in the compound of formula IV

BAD ORIGINAL

will be a 5-phenyl group. A 4-acetyl group may be introduced in this phenyl group by conventional Friedel-Crafts acylation. This acetyl group many subsequently be converted to an acetyloxy group by a treatment with trifluoroacetic acid and hydrogen peroxide. The acetyloxy group may in turn be hydrolysed, e.g. using potassium hydroxide in ethanol, to a hydroxy group. The hydroxy group may be converted to an alkoxy (e.g. methoxy) group by treatment with the appropriate alkyl iodide in basic conditions, e.g. in the presence of potassium carbonate. Similarly, the acetyl group may be converted to a 1-hydroxyethyl group by appropriate reduction, e.g. using sodium borohydride in ethanol, or it may be converted to an acetamido group by reaction with hydroxylamine followed by Beckmann rearrangement of the resulting (1-hydroxyimino)ethyl derivative. The acetamido group may in turn be hydrolysed in acid conditions, e.g. in the presence of hydrochloric acid, to an amino group.

Reduction with sodium amalgam is conveniently effected according to the method of Fredga, Acta Chem. Scand., 9, 719 (1955). The reduction may be effected after the $R^4$ group has been hydrolysed to a hydroxy group, or the reduction may be effected with hydrolysis in a single reaction sequence, to give a 2,3-dihydrobenzofurancarboxylic acid which, if desired, may subsequently be esterified in known manner.

In general, the $R^4$ group may be hydrolysed to a hydroxy group in known manner by base-catalysed hydrolysis using for example sodium or potassium hydroxide, conveniently in ethanol as solvent. The hydroxy group may then be esterified in known manner to give an $R^3$ group which is alkoxy of 1 to 4 carbon atoms or a 2-propenyloxy group, e.g. by Fisher-Speier esterification of the acid with the appropriate alcohol, i.e. by treating the acid with the alcohol using the alcohol itself as solvent, or using a solvent such as benzene or toluene, in the presence of a catalytic amount of an acid such as sulfuric acid, hydrochloric acid or para-toluenesulfonic acid.

It will be appreciated that by the above methods there may readily be prepared 2,3-dihydrobenzofurancarboxylic acid esters of formula

BAD ORIGINAL

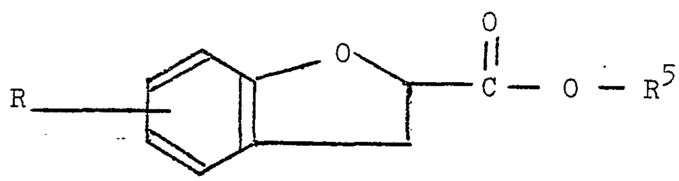

(V)

where R is as defined above and $R^5$ is an alkyl group. When $R^5$ is an alkyl group of 1 to 4 carbon atoms, the compounds of formula V are compounds of formula I. Compounds of formula V are intermediates in the preparation of compounds of formula I wherein $R^3$ is a 2-propenylamino group.

According to this aspect of the invention there is provided a process for preparing a benzofurancarboxylic acid derivative of formula I wherein $R^1$ and $R^2$ represent hydrogen atoms and $R^3$ represents a 2-propenylamino group, which process comprises reacting a 2,3-dihydro-2-benzofurancarboxylic acid ester derivative of formula V with 2-propenylamine.

Conveniently the process of this aspect of the invention is effected in the presence of an alkanol of one to four carbon atoms as solvent, and $R^5$ is an alkyl group of from 1 to 4 carbon atoms. In a preferred process, $R^5$ is ethyl and the process is effected using ethanol as solvent. The reaction will go forward at room temperature; however, higher temperatures -- for example, the mixture can be refluxed -- may be employed to reduce the reaction time. Preferably, about a four-to-six fold excess of the 2-propenamine is used. The desired product can be recovered by evaporating the solvent and excess amine, then employing conventional techniques such as selective extraction, recrystallization and/or dry column chromatography, to isolate the desired product.

Compounds of formula I have exhibited lipogenesis inhibiting activity in mammalian tissue.

Chirality exists in the compounds of formula I wherein $R^1$ and $R^2$ are hydrogen atoms, hence they can exist in two optical isomeric forms. None of the isomers has been separated, nor has the lipogenesis inhibiting activity of any of the individual isomers been determined. The individual species that have been prepared inhibit lipogenesis. Under the circumstances, the

invention contemplates the active individual isomers, as well as mixtures thereof.

Compounds of formula I can be used to inhibit lipogenesis in mammals such as, for example, pets, animals in zoo, livestock, furbearing animals and domestic animals, including, but not limited to dogs, cats, mink, sheep, swine, cattle, horses, mules and donkeys. The effect is obtained by administering an effective amount of one or a mixture of the inhibitors orally or parenterally to the animal. They may be administered as such, or as an active ingredient of a conventional pharmaceutical formulation. They may be administered orally by any convenient means. Thus, they may be orally administered as a drench, by intubation, in the animal's food and water, in a food supplement or in a formulation expressly designed for administration of the drug. Suitable formulations include solutions, suspensions, dispersions, emulsions, tablets, boluses, powders, granules, capsules, syrups and elixirs. For parenteral administration, they may be in the form of a solution, suspension, dispersion or emulsion. They can be administered in the form of an implant or other controlled sustained release formulation. Inert carriers, such as one or more of water, edible oil, gelatin, lactose, starch, magnesium stearate, talc or vegetable gum can be used. The dosage of the inhibitor needed to inhibit lipogenesis will depend upon the particular compound(s) used, and the particular animal being treated. However, in general, satisfactory results are obtained when the inhibitor is administered in a dosage of from about 1 to about 500 milligrams per kilogram of the amimal's body weight. The inhibitor can be administered in a single dose or in a series of doses in the same day, or over a period of days. For any particular animal, a specified dosage regium should be adjusted according to the individual need, the particular inhibitor used, and the professional judgement of the person administering or supervising the administration of the inhibitor.

Accordingly the invention also provides a lipogenesis inhibiting composition comprising a compound of formula I in association with a pharmaceutically or veterinarily acceptable

carrier therefor. Further provided is a process for preparing a lipogenesis inhibiting composition which comprises bringing a compound of formula I into association with pharmaceutically or veterinarily acceptable carrier.

The invention also includes a method of inhibiting lipogenesis in a mammal which comprises administering to the mammal orally or parenterally a compound of formula I or a composition according to the invention.

The invention will be further understood from the following examples, in each of which the identities of the products, and the intermediates involved, were confirmed by appropriate elemental and spectral analyses.

Example 1 Ethyl 4-phenoxybenzofuran-2-carboxylate (1)

145.0 g of meta-phenoxyphenol was dissolved in 700 ml of 95% ethanol. 468 g of sodium hydroxide was then added rapidly. The resulting suspension was heated to 70-80°C. Then 558.7 g of chloroform was added, at such a rate that gentle reflux was maintained (the addition required 10 hours). The mixture then was stirred for 2 hours at 75-80°C, held at room temperature overnight and then was filtered. The solid product was dissolved in 1000 ml of water. The solution was acidified to pH=2 with concentrated hydrochloric acid, then was extracted with ether. The ether layer was dried ($MgSO_4$) and concentrated. The residue was extracted with hot petroleum ether. The extract was dried ($Na_2SO_4$) and concentrated to give an oil, which was wet column chromatographed over silica gel, using a 9/1 v/v mixture of petroleum ether and ether as eluent, then using a 4/1 v/v mixture of petroleum ether and ether as eluent. The fourth fraction obtained was identified as 2-hydroxy-6-phenoxybenzaldehyde (1A).

A mixture of 1.07 g of 1A, 0.96 g of diethyl bromomalonate and 1.25 g of anhydrous potassium carbonate in 20 ml of 2-butanone, was refluxed for 10 hours. The solvent was evaporated under reduced pressure. The residue was cooled, poured into 100 ml of water and extracted with ether. The extract was washed with cold 5% sodium hyroxide solution and water and then concentrated under

BAD ORIGINAL

0019955

reduced pressure. The residue was recrystallized from ethanol to give ethyl 4-phenoxybenzofuran-2-carboxylate (1), as a liquid.

Example 2 4-Phenoxy-2-benzofurancarboxylic acid (2)

A mixture of 1.4 g of compound 1 and 50 ml of 10% alcoholic potassium hydroxide was refluxed for 4 hours. The solvent was evaporated under reduced pressure and the residue was washed with ether and dissolved in water. The basic solution was acidified with dilute hydrochloric acid and extracted with ether. The ether layer was extracted with dilute sodium bicarbonate solution. The aqueous solution was re-acidified with dilute hydrochloric acid and extracted with ether. The ether extract was dried ($Na_2SO_4$) and concentrated under reduced pressure. The residue was crystallized from ethanol to give 4-phenoxy-2-benzofurancarboxylic acid (2). mp: 215-217°C.

Example 3 2,3-Dihydro-4-phenoxy-2-benzofurancarboxylic acid (3)

5.3 g of 2 was mixed with 90 ml of 10% of sodium hydroxide solution. Sodium amalgam (prepared from 1.5 g of sodium and 50 g of mercury) was added to the stirred mixture over a period of one hour. The mixture was then stirred for 24 hours and allowed to stand at room temperature for an additional 24 hours. The mercury was separated, the solution was neutralized with dilute hydrochloric acid and extracted with ether. The extract was dried ($Na_2SO_4$) and concentrated under reduced pressure. The residue was recrystallized from ethanol to give 2,3-dihydro-4-phenoxy-2-benzofurancarboxylic acid (3), mp: 123-125°C.

Example 4 Ethyl 2,3-dihydro-4-phenoxy-2-benzofurancarboxylate (4)

A mixture of 2.7 g of 3, 60 ml of ethanol, 20 ml of dry benzene and 2 ml of concentrated sulfuric acid was refluxed for seven hours, with removal of water as it formed. The resulting mixture was concentrated, and the residue dissolved in ether. The solution was washed with saturated sodium bicarbonate solution and water. The aqueous portion was extracted with ether. The combined organic layers were dried ($Na_2SO_4$) and the solvent was evaporated under reduced pressure. The residue was distilled to give ethyl 2,3-dihydro-4-phenoxybenzofuran-2-carboxylate (4), mp: 56-58°C.

BAD ORIGINAL

Example 5 2,3-Dihydro-4-phenoxy-N-(2-propenyl)-2-benzofuran-carboxamide (5)

An ethanol solution of 4 and an excess of 2-propenamine was refluxed for 2.5 days. The excess amine and the solvent were evaporated under reduced pressure. The resulting gum was recrystallized from methylene chloride/hexane to give 5, mp: 55-57°C.

Example 6 Ethyl 5-(4-acetylphenyl)benzofuran-2-carboxylate (6)

12.2 g of acetyl chloride was added to a mixture of 10.3 g of ethyl 5-phenylbenzofuran-2-carboxylate (Witiak, D.T., et al, Lipids, 11 (1976) (5) 394-391 and printed U.K. Patent Application No 2 007 973), and 120 ml of carbon disulfide, then 21.8 g of anhydrous aluminium chloride was added in portions to the stirred mixture. The mixture then was stirred at room temperature for 2.5 hours, the temperature rising to 32°C. The mixture was poured into 1 liter of ice water and stirred for 30 minutes, and the solution was extracted with ether. The extract was dried ($MgSO_4$) and concentrated. The residue was washed with ether and dissolved in 100 ml of chloroform. The solution was treated with activated charcoal and 100 ml of hexane and added. The resulting solution was concentrated to about 100 ml and cooled to give 6, mp: 105-107°C.

Example 7 Ethyl 5-(4-acetyloxyphenyl)benzofuran-2-carboxylate (7)

2 ml of trifluoroacetic acid was added to a mixture of 30.8 g of 6 and 500 ml of acetic acid, then 100 ml of 30% hydrogen peroxide solution was added drop-by-drop. The mixture was stirred over a weekend at room temperature, then heated at 55-60°C for 8 hours and allowed to stand overnight. The resulting solid was collected, dried under reduced pressure, recrystallized from 2/3 v/v methylene chloride/hexane mixture to give 7, mp: 143-145°C.

Example 8 5-(4-Acetylphenyl)-2-benzofurancarboxylic acid (8)

6 was treated with potassium hydroxide in ethanol to give 8, mp: 264-266°C.

Example 9 5-(4-(Acetyloxy)phenyl)-2-benzofurancarboxylic acid (9)

2 ml of trifluoroacetic acid and 92 ml of 30% hydrogen peroxide solution were added to a mixture of 13 g of 8 in 300 ml of acetic acid. The mixture was heated at 70-75°C for 10 hours,

BAD ORIGINAL

then was cooled in a refrigerator. The solid that formed was collected and dried. The acetic acid solution was poured into 3.6 liters of ice water. The solid that formed was collected and dried ($P_2O_5$). The two solids were combined and dissolved in 150 ml of tetrahydrofuran. The solution was filtered, concentrated to 20 ml and chilled. The solid that formed was collected, dried under reduced pressure, and recrystallized from tetrahydrofuran to give 9, mp: 251-254°C.

Example 10 5-(4-Hydroxyphenyl)-2-benzofurancarboxylic acid (10)

A mixture of 10.4 g of 9 and 7.2 g of potassium hydroxide in 170 ml of ethanol was refluxed for 2 hours. The solvent was evaporated under reduced pressure. The residue was mixed with 200 ml of water, and the mixture was acidified to pH=2 with concentrated hydrochloric acid, stirred for 30 minutes and filtered. The solid was washed with water, dried under reduced pressure and mixed with tetrahydrofuran. The mixture was refluxed and filtered. The filtrate was concentrated, the residue was washed with ether and mixed with tetrahydrofuran. The mixture was filtered, the filtrate was concentrated to about one-fourth its volume and triturated with ether. The resulting solid was collected and dried under reduced pressure to give 10, mp: 297-300°C.

Example 11 5-(4-Methoxyphenyl)-2-benzofurancarboxylic acid (11)

16.6 g of methyl iodide and 17.8 g of potassium carbonate were added to a mixture of 20.0 g of 10, 400 ml of dimethyl sulfoxide and 150 ml of acetone. The mixture was heated at 65-70°C for 3 hours. A further 16.5 g of methyl iodide and 8.9 g of potassium carbonate were added, and the heating was continued for 4 hours. The mixture was poured into ice water. The resulting solid was collected, washed with water and dried ($P_2O_5$, reduced pressure). The solid was mixed with tetrahydrofuran, the mixture was filtered, the filtrate was dried ($Na_2SO_4$) and concentrated to a small volume. The resulting solid was separated and recrystallized from 2/1 v/v mixture of chloroform and hexane. The product was dry column chromatographed, using chloroform as eluent. The solvent was evaporated, the appropriate fractions were extracted

BAD ORIGINAL

with chloroform and the solvent was evaporated from the extract. The residue was washed with ether and dried under reduced pressure to give methyl 5-(4-methoxyphenyl)-2-benzofurancarboxlate, 11A, mp: 160-162°C.

11A was treated with potassium hydroxide to give 11, mp: 181-184°C.

Example 12 Ethyl 5-(4-(1-hydroxyethyl)phenyl)benzofuran-2-carboxylate (12)

1.2 g of sodium borohydride was added in one portion to a stirred mixture of 15.0 g of 6, 200 ml of ether and 40 ml of ethanol. The mixture was stirred at room temperature for 2 hours. The solvents were evaporated under reduced pressure. 1.6 litres of water was added to the residue and the mixture was extracted with chloroform. The extract was dried ($MgSO_4$) and the solvent was evaporated under reduced pressure. The residue was dissolved in 150 ml of chloroform. The solution was filtered through charcoal. 400 ml of hexane was added to the filtrate and the solid which formed was collected, recrystallized from a 3/50 v/v mixture of chloroform and hexane and dried in a vacuum to give 12, mp: 108-109°C.

Example 13 Methyl 2,3-dihydro-5-(4-(1-hydroxethyl)phenyl-2-benzofurancarboxylate (13)

75 ml of ethanol was added to a solution of 23.9 g of 12 in 300 ml of tetrahydrofuran, then 195.7 g of 2.5% sodium/mercury amalgam was added in portions, at room temperature. The mixture was stirred at room temperature overnight. The mercury was separated, the solid was collected and disolved in 300 ml of water. The solution was acidified to pH=1 with concentrated hydrochloric acid. The solid was collected, washed with water and dried ($P_2O_5$; reduced pressure, 45°C). The product was dissolved in tetrahydrofuran, the solution was filtered and its volume reduced to 30 ml by evaporating the solvent under reduced pressure. The resulting solution was triturated with ether. The solid was collected and dried in a vacuum oven over $P_2O_5$ to give 2,3-dihydro-5-(4-(1-hydroxyethyl)phenyl)-2-benzofurancarboxylic acid (13A), mp: 155-157°C.

BAD ORIGINAL

23.3 g of methyl iodide and 7.6 g of potassium carbonate were added to a mixture of 7.8 g of 13A, 200 ml of acetone and 50 ml of dimethylsulfoxide. The resulting suspension was heated under reflux for 2 hours. The mixture was filtered and the filtrate was concentrated under reduced pressure. The residue was dissolved in tetrahydrofuran. The solution was filtered and the filtrate was concentrated. The residue was mixed with 50 g of silica gel and purified by dry column chromatography, a 9/1 v/v chloroform/ tetrahydrofuran mixture being used as eluent. The band containing product was extracted with tetrahydrofuran, the solvent was evaporated from the extract under reduced pressure and the residue was dried in a vacuum oven. The residue was stirred with ether and the solid was collected and dried to give methyl 2,3-dihydro-5-(4-(1-hydroxyethyl)phenyl)-2-benzofurancarboxylate 13, mp: 98-100°C.

Example 14 2,3-Dihydro-5-(4-(1-hydroxyethyl)phenyl)-N-(2-propenyl)-2-benzofurancarboxamide (14)

15 ml of 2-propenylamine was added to a mixture of 5.0 g of 13 and 100 ml of methanol. The solution was stirred at room temperature overnight. The volatile materials were evaporated under reduced pressure. The residue was stirred with 50 ml of ether, the solid was collected and dried in a vacuum oven to give 14, mp: 122-124°C.

Example 15 Ethyl 5-(4-(acetylamino)phenyl)furan-2-carboxylate (15)

A mixture of 6.2 g of 6, 100 ml of ethanol and 200 ml of tetrahydrofuran was heated to 60-70°C. A solution of 1.53 g of hydroxylamine hydrochloride and 1.16 g of sodium carbonate in 20 ml of water was added. The mixuture was heated for 3 hours at 60-70°C. The resulting solid was collected, washed with water, then ethanol, and extracted with methylene chloride. The solvent was evaporated from the extract under reduced pressure to give ethyl 5-(4-(1-(hydroxyimino)ethyl)phenyl-2-benzofurancarboxylate (15A), mp: 220-222°C.

56.5 g of phosphorus pentachloride was added in portions to a solution of 55.0 g of 15A in 1 liter of chloroform, and the mixture was stirred at room temperature for 16 hours. The

BAD ORIGINAL

solvent was evaporated under reduced pressure. The residue was suspended in 4 liters of water, the mixture was stirred vigorously for 30 minutes and filtered. The solid was extracted with chloroform. The extract was washed, successively, with water, saturated sodium bicarbonate solution, and water, then was filtered through celite (to break the emulsion). The filtrate was dried ($MgSO_4$) and the solvent was evaporated under reduced pressure. The residue was triturated with ether. The ether phase was separated, then concentrated to about half its volume under reduced pressure and allowed to stand over a weekend. The solid which formed was collected and dissolved in 400 ml of chloroform. The solution was filtered over charcoal and diluted with 400 ml of hexane. The resulting solid was collected and dry column chromatographed over silica gel, using a 1/9 v/v mixture of tetrahydrofuran and chloroform as eluent. After removal of the solvents, the appropriate fractions were combined and extracted with tetrahydrofuran. The solvent was evaporated and evaporated under reduced pressure, and the residue was triturated with ether. The resulting solid was collected and dried under reduced pressure to give 15, mp: 174-176°C.

Example 16 Ethyl 5-(4-aminophenyl)benzofuran-2-carboxylate (16)

A mixture of 3.0 g of 15, 50 ml of 6N hydrochloric acid and 50 ml of ethanol was stirred and refluxed for 5 hours. The solution was concentrated to half its volume, the resulting solid was collected and dissolved in chloroform containing some triethylamine. The solution was washed with water, dried ($MgSO_4$) was filtered. The solvent was evaporated from the filtrate under reduced pressure. The residue was dry column chromatographed using a 1/9 v/v tetrahydrofuran/chloroform mixture as eluent. The solvents were evaporated, the appropriate fractions were combined and extracted with chloroform. The extract was filtered and the solvent was evaporated under reduced pressure. The residue was refluxed in petroleum ether for 8 hours. The mixture was filtered and the filtrated was held in a freezer overnight. The resulting solid was collected and dried to give 16, mp: 95-97°C.

Example 17 Ethyl 5-(4-(acetylamino)phenyl)-2,3-dihydro-2-benzofurancarboxylate (17)

25 ml of ethanol was added to a mixture of 2.3 g of 15 in 80 ml of tetrahydrofuran. Then 18.8 g of 2.5% sodium amalgam was added in portions, and the mixture was stirred for 20 hours. The mercury was separated and the solvent was evaporated under reduced pressure. The residue was dissolved in 350 ml of water, the solution was filtered, and concentrated hydrochloric acid was added until the solution had a pH of 2. The solid was collected, washed with water and dried under reduced pressure. The residue was stirred with 30 ml of acetone. The solid was collected and refluxed with acetone. The solution was filtered and the filtrate was concentrated to a small volume under reduced pressure. The solid was collected and dried, to give 5-(4-(acetylamino)phenyl)-2-3-dihydro-2-benzofurancarboxylic acid (17A), mp: 274-276°C.

5 drops of concentrated sulfuric acid was added to a mixture of 3.2 g of 17A and 150 ml of ethanol. The mixture was heated under reflux, using a Soxhlet extractor filled with molecular sieve (3A). After 4 hours, the solvent was evaporated under reduced pressure, and the residue was dissolved in chloroform. The solution was washed with saturated sodium bicarbonate solution, dried ($MgSO_4$) and the solvent was evaporated under reduced pressure. The residue was dry column chromatographed over silica gel, using chloroform as eluent. The product was extracted with tetrahydrofuran and the solvent was evaporated under reduced pressure. The residue was stirred in ether. The solid was collected and refluxed in ether. The solid was collected and dried under reduced pressure to give ethyl 5-(4-(acetylamino)-2,3-dihydro-2-benzofurancarboxylate (17), mp: 164-166°C.

Example 18 5-(4-(Acetylamino)phenyl)-2,3-dihydro-N-(2-propenyl)-2-benzofurancarboxamide (18)

20 ml of ethanol was added to a mixture of 1.6 g of 17 and 20 ml of 2-propenylamine. The mixture was stirred for 3 hours at room temperature. The solid which formed was collected, washed with ethanol, then ether and dried under reduced pressure to give 18, mp: 244-247°C.

BAD ORIGINAL

Compounds of formula I have been found to inhibit lipogenesis in tissues of mammals. The manner in which they cause this effect is not known with certainty; it is believed that they interfere with the synthesis of fatty acids in the tissues. Their effectiveness for this purpose has been ascertained by immersing samples of swine adipose tissue in a liquid medium containing radioactive glucose and the test chemical for a period of time, the isolating the lipid from the treated tissue and determining the uptake of the radioactive carbon by means of scintillation counting techniques. These tests were conducted in swine adipose tissue because in swine, the primary site of lipogenesis -- i.e., fatty acid synthesis -- appears to be adipose tissue.

Described in more detail, the tests were conducted according to the following general procedure:

150 milligrams of slices of swine adipose tissue were incubated at 37°C for 2 hours with shaking in 3 milliliters of Krebs-Ringer bicarbonate solution containing one-half of the normal calcium ion concentration, 60 micromoles of glucose, 0.5 micro-Curie of glucose-$U^{14}C$, and 300 micounits of insulin, and 5% dimethyl sulfoxide (DMSO). The test compound was added as a solution or suspension in DMSO and was present as a concentration of 100 micrograms per milliliter of incubation mixture.

The incubation was terminated by addition of 0.25 milliliter of 1 N sulfuric acid. The resulting mixture was extracted with total of 25 milliliters of chloroform/methanol (2:1, v/v). The extracts were washed according to Folch et al. (J. Biol. Chem., <u>226</u>, 497-509, (1957)), air dried, and counted in a liquid scintillation countre with 15 milliliters of counting fluid (two parts toulene containing 0.4% w/v New England Nuclear Omnifluor: 1 part Triton X-100). The tests were conducted in triplicate and were accompanied by control tests in which all ingredients, proportions and conditions were the same except that no test compound was included. From the data obtained were calculated the percent inhibition of lipid synthesis by the test compound. The data obtained are reported as the percent inhibition of

lipogenesis compared to the results obtained in the control tests wherein only the test compound was omitted. The results are summarized in Table I.

Table I

| Compound | Percent Inhibition |
|---|---|
| 1 | 28 |
| 2 | 83 |
| 3 | 27 |
| 4 | 20 |
| 5 | 77 |
| 6 | 24 |
| 7 | 56 |
| 8 | 41 |
| 9 | 80 |
| 10 | 30 |
| 11 | 56 |
| 12 | 71 |
| 13 | 85 |
| 14 | 40 |
| 15 | 52 |
| 16 | 45 |
| 17 | 88 |
| 18 | 76 |

CLAIMS

1. A benzofurancarboxylic acid derivative of formula

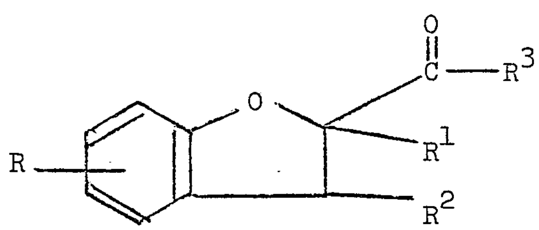

(I)

wherein R is a substituent at the 5-position on the benzofuran ring system and is a thienyl group, or a phenyl, phenoxy, benzyl or benzoyl group substituted by a group selected from alkoxy of 1 to 6 carbon atoms, hydroxy, acetyl, acetyloxy, 1-hydroxyethyl, acetamido and amino, or is a substituent at the 4-position of the benzofuran ring system and is a phenoxy group optionally substituted by a group selected from alkyl and alkoxy of 1 to 6 carbon atoms, hydroxy, acetyl, acetyloxy, 1-hydroxyethyl, acetamido, amino, fluoro, chloro, bromo and nitro; $R^1$ and $R^2$ represent hydrogen atoms or $R^1$ and $R^2$ together represent a single chemical bond; and $R^3$ represents a hydroxy group, an alkoxy group of from 1 to 4 carbon atoms or a 2-propenyloxy group, or, when $R^1$ and $R^2$ represent hydrogen atoms, a 2-propenylamino group.

2. A derivative according to Claim 1 <u>further characterised in that</u> R is a substituent at the 5-position on the benzofuran ring system and is a phenyl group substituted at the 4-position by an acetyl, acetyloxy, hydroxy, methoxy, 1-hydroxyethyl, acetamido or amino group, or is a phenoxy group attached at the 4-position on the benzofuran ring system.

3. A derivative according to claim 1 or 2 further characterised in that R is a 5-(4-acetyloxyphenyl), 5-(4-(1-hydroxyethyl)-phenyl), 5-(4-acetamidophenyl) or 4-phenoxy group.

4. A derivative according to any one of Claims 1 to 3 further characterised in that $R^3$ represents a hydroxy, methoxy or ethoxy group or, when $R^1$ and $R^2$ represent hydrogen atoms, a 2-propenylamino group.

5. A derivative according to any one of Claims 1 to 4 further characterised in that $R^3$ is hydroxy, $R^1$ and $R^2$ together represents a single chemical bond and R is 4-phenoxy or 5-(4-acetyloxyphenyl); $R^3$ is methoxy or ethoxy and R is 5-(4-(1-hydroxyethyl)phenyl) or 5-(4-acetamidophenyl); or $R^3$ is 2-propenylamino and R is 4-phenoxy or 5-(4-acetamidophenyl).

6. A process for preparing a benzofurancarboxylic acid derivative of formula I as defined in any one of Claims 1 to 5, which process is characterised by reacting a salicylaldehyde of formula

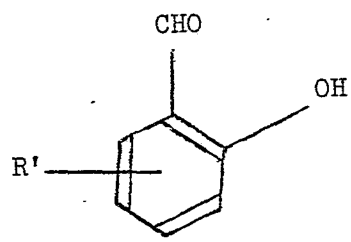

(II)

where R' is attached at the 5- or 6- position of the benzene ring and is R as defined in Claim 1 or a group which is convertible to R, with a halmalonate ester of formula

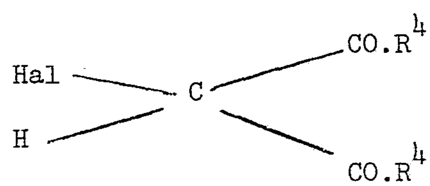

(III)

where Hal is bromine or chlorine and each $R^4$ is alkoxy of 1 to 4 carbon atoms in the presence of a base to give a benzofurancarboxylate ester of formula

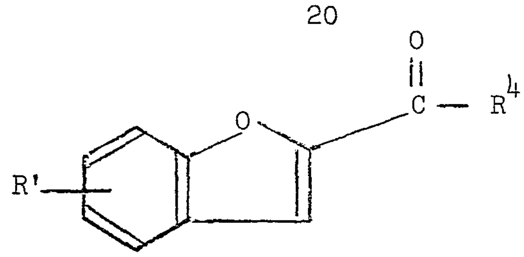

(IV)

followed, where necessary, by conversion of R' to R, reduction with sodium amalgam and/or conversion of $R^4$ to $R^3$.

7. A process for preparing a benzofurancarboxylic acid derivative of formula I wherein $R^1$ and $R^2$ represent hydrogen atoms, $R^3$ represents a 2-propenylamino group and R is as defined in any one of Claims 1 to 5, which process is <u>characterised by</u> reacting a 2,3-dihydro-2-benzofurancarboxlic acid ester derivative of formula

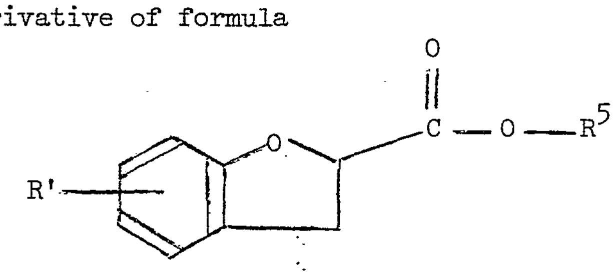

(V)

wherein R is as defined in Claim 1 and $R^5$ is an alkyl group, with 2-propenylamine.

8. A lipogenesis inhibiting composition comprising a compound of formula I as defined in any one Claims 1 to 5 in association with a pharamaceutically or veterinarily-acceptable carrier therefor.

9. A process for preparing a lipogenesis inhibiting composition which comprises bringing a compound of formula I as defined in any one of Claims 1 to 5 into association with a pharaceutically or veterinarily-acceptable carrier.

0019955

Application number

European Patent Office

# EUROPEAN SEARCH REPORT

EP 80 20 0412.7

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.3) |
|---|---|---|---|
| D P | GB - A - 2 007 973 (SHELL INTERNATIONALE RESEARCH MAATSCHAPPIJ)<br>* claims 1, 8, 11, 15 *<br>-- | 1,6-8 | C 07 D 307/85<br>A 61 K 31/34 |
| | DE - A1 - 2 811 236 (SHELL INTERNATIONALE RESEARCH MAATSCHAPPIJ)<br>* claim 1 *<br>-- | 8 | |
| | Chemical Abstracts vol. 85, no. 8, 23 August 1976<br>Columbus, Ohio, USA<br>D.T. WITIAK et al."Comparative antilipidemic effects of various ethyl 5-substituted benzofuran-, 2,3-dihydrobenzofuran- and 3(2H)-benzofuranone-2-carboxylate analogs of clofibrate in a Triton hyperlipidemic rat model"<br>page 18, column 2, abstract No. 56541z<br>& Lipids, vol. 11, No. 5, 1976, pages 384 to 391<br>-- | 1,8 | TECHNICAL FIELDS SEARCHED (Int.Cl.3)<br>A 61 K 31/34<br>C 07 D 307/85 |
| P | US - A - 4 178 380 (SHELL OIL COMPANY)<br>* abstract *<br>--<br>./.. | 8 | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons<br>&: member of the same patent family, corresponding document |
| X | The present search report has been drawn up for all claims | | |

| Place of search | Date of completion of the search | Examiner |
|---|---|---|
| Berlin | 02-09-1980 | FROELICH |

EPO Form 1503.1 06.78

0019955

European Patent Office

# EUROPEAN SEARCH REPORT

Application number
EP 80 20 0412.7
- page 2 -

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| A | DE - A1 - 2 630 800 (MERCK & CO.)<br>-- | | |
| A | DE - A1 - 2 754 068 (MERCK & CO.)<br>---- | | |
| | | | TECHNICAL FIELDS SEARCHED (Int. Cl.3) |